# EUROPEAN PATENT SPECIFICATION

(11) **EP 0 789 199 B1**
(45) Date of publication and mention of the grant of the patent: **07.05.2003**
(21) Application number: 97101886.6
(22) Date of filing: 06.02.1997
(51) Int. Cl.: F24F 3/14, F25B 43/00, B01D 15/00, F24F 3/06

(54) **Air conditioner**
Klimaanlage
Installation de conditionnement d'air

(30) Priority: 09.02.1996 JP 2358096
(43) Date of publication of application: 13.08.1997
(60) Divisional application: 02010392.5
(73) Proprietor: MATSUSHITA ELECTRIC INDUSTRIAL CO., LTD., Kadoma-shi, Osaka-fu (JP)
(72) Inventor: Otaki, Shizuo, Otsu-shi, Shiga (JP)
(74) Representative: Eisenführ, Speiser & Partner

(56) References cited:
- DE-A- 3 601 342
- DE-A- 4 238 853
- DE-A- 4 319 293

## Description

### BACKGROUND OF THE INVENTION

### (Field of the Invention)

The present invention relates to an air conditioner provided with a refrigerating cycle having a dryer.

### (Description of Related Art)

DE-A-42 38 353 representing the closest prior art from which the invention proceeds discloses a condenser for an air conditioning device in a motor vehicle wherein a collector is connected to a condensing section via a first connection aperture and to an undercooling section via a second connection aperture in a base area. A collection tube and the parallel collector are formed as a double tube wherein the apertures are located in the dividing wall between the chambers of the double tube. A dryer is integrated in the condensing section in the form of the double wall construction.

In DE-A-43 19 293, it is described an air conditioning system for a car which system comprises a condenser made of finned tubes in a block and terminating in pipes at either side. The collector is a one-piece unit with these pipes. A dryer is provided having a matrix containing little sacks of granulate through which a coolant can percolate. The upper end of the collector has a detachable lid whose opening is larger than the cross-section of the dryer. The lid is screwed down and contains a pressure switch. The dryer is supported inside the collector by elastic spacers.

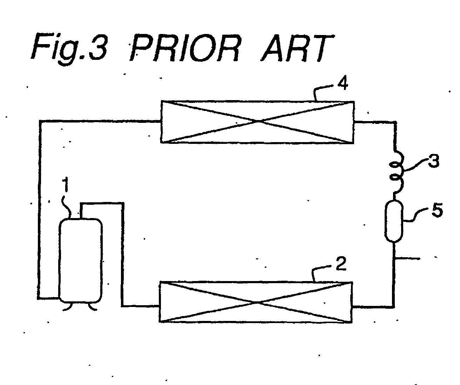

Fig. 3 depicts a further conventional refrigerating cycle comprising a compressor 1, a condenser 2, a throttling device 3, an evaporator 4, and a dryer 5, all of which are connected in series. As shown in Fig. 3, the dryer 5 is interposed between the condenser 2 and the throttling device 3.

In the above-described construction, however, when the refrigerating cycle is in operation, all refrigerant passes through a drying agent accommodated in the dryer 5. Because of this, a refrigerant flow vibrates and wears away particles of the drying agent, and the resultant worn-out powder tends to adhere to the throttling device 3 or the compressor 1, resulting in excessive throttling or poor compression.

### SUMMARY OF THE INVENTION

The present invention has been developed to overcome the above-described disadvantages.

It is accordingly an objective of the present invention to provide an air conditioner capable of preventing wear of the drying agent which has been hitherto caused by a vibration of agent particles resulting from a refrigerant flow.

In accomplishing the above and other objects, in accordance with a first aspect of the present invention there is provided an air conditioner provided with a refrigerating cycle having a compressor, a condenser, a throttling device, an evaporator connected in series by a plurality of connecting pipes, and a dryer connected to said refrigerating cycle, characterized in that said dryer is connected to one of said plurality of connecting pipes in parallel therewith.

By the above-described construction, a small amount of refrigerant flows through the dryer connected in parallel with a main refrigerant passage at a relatively low speed. Accordingly, a drying agent accommodated in the dryer can effectively adsorb moisture contained in the refrigerant while preventing wear of particles of the drying agent.

In accordance with a second aspect of the present invention, there is provided an air conditioner provided with a refrigerating cycle having a compressor, a condenser, a throttling device, an evaporator connected in series by a plurality of connecting pipes, and a dryer connected on one side thereof to one of said plurality of connecting pipes, characterized in that said dryer is connected on the other side thereof to a filling pipe through which the refrigerating cycle is charged with refrigerant.

According to this construction, because the refrigerant does not directly pass through the dryer, wear of particles of the drying agent is avoided. However, because the dryer communicates with the circulating refrigerant, the drying agent in the dryer can adsorb moisture contained in the refrigerant and entering the dryer by diffusion. Furthermore, during filling of the refrigerant into the refrigerating cycle, the drying agent can adsorb moisture in the refrigerant.

### BRIEF DESCRIPTION OF THE DRAWINGS

The above and other objectives and features of the present invention will become more apparent from the following description of preferred embodiments thereof with reference to the accompanying drawings, throughout which like parts are designated by like reference numerals, and wherein:
Fig. 1 is a schematic diagram of a refrigerating cycle employed in an air conditioner according to a first embodiment of the present invention;
Fig. 2 is a diagram similar to Fig. 1, but according to a second embodiment of the present invention; and
Fig. 3 is a schematic diagram of a refrigerating cycle employed in a conventional air conditioner.

### DETAILED DESCRIPTION OF THE PREFERRED EMBODIMENTS

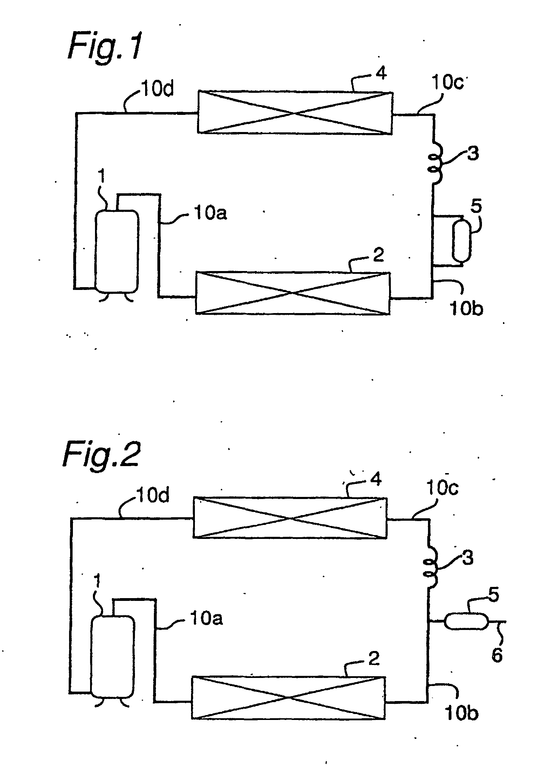

Referring now to the drawings, there is schematically shown in Fig. 1 a refrigerating cycle employed in an air conditioner according to a first embodiment of the present invention, which comprises a compressor 1, a condenser 2 connected to the compressor 1 via a connecting pipe 10a, a throttling device 3 connected to the condenser 2 via a connecting pipe 10b, and an evaporator 4 connected to the throttling device 3 via a connecting pipe 10c and to the compressor 1 via a connecting pipe 10d. The refrigerating cycle shown in Fig. 1 also comprises a dryer 5 connected to the connecting pipe 10b in parallel therewith.

In the refrigerating cycle of the above-described construction, when the compressor 1 is in operation, refrigerant in the compressor 1 circulates through the condenser 2, the throttling device 3, and the evaporator 4 in this order and returns to the compressor 1. Because the connecting pipe 10b connecting the condenser 2 and the throttling device 3 is a mere pipe and because the dryer 5 accommodates a drying agent, a net or meshes encircling the drying agent, and the like, most of the refrigerant flows through the connecting pipe 10b, while a small amount of refrigerant flows through the dryer 5. Because of this, the speed at which the refrigerant flows through the dryer 5 is relatively low and, hence, the refrigerant does not vibrate the drying agent in the dryer 5, thus avoiding wear of particles of the drying agent.

Fig. 2 depicts a refrigerating cycle employed in an air conditioner according to a second embodiment of the present invention. As shown therein, the dryer 5 is connected on its one side to the connecting pipe 10b connecting the condenser 2 and the throttling device 3 and on its other side to a filling pipe 6 through which the refrigerating cycle is charged with the refrigerant.

According to the above-described construction, the refrigerant does not pass through the dryer 5 and, hence, does not vibrate the drying agent in the dryer 5. However, because the dryer 5 communicates with the circulating refrigerant, the drying agent in the dryer 5 can capture or adsorb moisture contained in the refrigerant in the refrigerating cycle, which moisture enters the dryer 5 by diffusion regardless of whether the refrigerating cycle is in operation. Furthermore, when the refrigerant is injected into the refrigerating cycle, the refrigerant inevitably passes through the dryer 5 and, hence, the drying agent in the dryer 5 adsorbs moisture contained in the refrigerant.

It is to be noted here that although in the above first and second embodiments, the refrigerating cycle has been described as being of a unidirectional type allowing the refrigerant to flow in only one direction, similar effects can be obtained in a reversible type refrigerating cycle employing a four-way valve or the like.

## Claims

1. An air conditioner provided with a refrigerating cycle having a compressor (1), a condenser (2), a throttling device (3), an evaporator (4) connected in series by a plurality of connecting pipes (10a, 10b, 10c, 10d), and a dryer (5) connected to said refrigerating cycle,
**characterized in that** said dryer (5) is connected to one of said plurality of connecting pipes (10a, 10b, 10c, 10d) in parallel therewith.

2. An air conditioner provided with a refrigerating cycle having a compressor (1), a condenser (2), a throttling device (3), an evaporator (4) connected in series by a plurality of connecting pipes (10a, 10b, 10c, 10d), and a dryer (5) connected on one side thereof to one of said plurality of connecting pipes (10a, 10b, 10c, 10d),
**characterized in that** said dryer (5) is connected on the other side thereof to a filling pipe (6) through which the refrigerating cycle is charged with refrigerant.

## Patentansprüche

1. Klimaanlage, die mit einem Kühlkreislauf versehen ist, der einen Kompressor (1), einen Kondensator (2), eine Drosseleinrichtung (3) und einen Verdampfer (4) aufweist, die in Reihe durch mehrere Verbindungsrohre (10a, 10b, 10c, 10d) verbunden sind, und der weiterhin einen Trockner (5) enthält, welcher mit dem Kühlkreislauf verbunden ist,
**dadurch gekennzeichnet, dass** der Trockner (5) mit einem der mehreren Verbindungsrohre (10a, 10b, 10c, 10d) in paralleler Anordnung zu diesem verbunden ist.

2. Klimaanlage, die mit einem Kühlkreislauf versehen ist, der einen Kompressor (1), einen Kondensator (2), eine Drosseleinrichtung (3) und einen Verdampfer (4) aufweist, die in Reihe durch mehrere Verbindungsrohre (10a, 10b, 10c, 10d) verbunden sind, und der weiterhin einen Trockner (5) enthält, welcher an seiner einen Seite mit einem der mehreren Verbindungsrohre (10a, 10b, 10c, 10d) verbunden ist,
**dadurch gekennzeichnet, dass** der Trockner (5) an der anderen Seite mit einem Füllrohr (6) verbunden ist, durch das der Kühlkreislauf mit Kühlmittel beladen wird.

## Revendications

1. Une installation de conditionnement d'air prévue avec un cycle frigorifique comportant un compresseur (1), un condenseur (2), un organe d'étranglement (3), un évaporateur (4) reliés en série par une pluralité de tuyaux de raccordement (10a, 10b, 10c, 10d), et un sécheur (5) relié audit cycle frigorifique,
**caractérisée en ce que** ledit sécheur (5) est relié en parallèle à ladite pluralité de tuyaux de raccordement (10a, 10b, 10c, 10d).

2. Une installation de conditionnement d'air prévue avec un cycle frigorifique comportant un compresseur (1), un condenseur (2), un organe d'étranglement (3), un évaporateur (4) reliés en série par une pluralité de tuyaux de raccordement (10a, 10b, 10c, 10d), et un sécheur (5) relié en un de ses côtés à ladite pluralité de tuyaux de raccordement (10a, 10b, 10c, 10d),
**caractérisée en ce que** ledit sécheur (5) est relié en son autre côté à un tuyau de remplissage (6) par lequel le cycle frigorifique est alimenté en fluide frigorigène.
